# EUROPÄISCHE PATENTSCHRIFT

(11) **EP 1 059 065 B1**
(45) Veröffentlichungstag und Bekanntmachung des Hinweises auf die Patenterteilung: **07.04.2004**
(21) Anmeldenummer: 00110731.7
(22) Anmeldetag: 19.05.2000
(51) Int. Cl.: A61B 6/00, A61B 6/04

(54) **Röntgenuntersuchungsausrüstung**
Equipment for X-ray examination
Equipement pour la radiographie

(30) Priorität: 10.06.1999 SE 9902181
(43) Veröffentlichungstag der Anmeldung: 13.12.2000
(73) Patentinhaber: SIEMENS AKTIENGESELLSCHAFT, 80333 München (DE)
(72) Erfinder: Mellström, Erik, 175 65 Järfälla (SE); Stenfors, Per, 163 44 Spanga (SE); Narfström, Jan, 192 51 Sollentuna (SE)

(56) Entgegenhaltungen:
- FR-A- 2 581 856
- US-A- 4 019 059
- Prospekt: " Der neue Massstab für Multifunktionsanlagen Polystar T.O.P."; Fa. Siemens

## Beschreibung

Die Erfindung betrifft eine Röntgenuntersuchungsausrüstung mit einem Fundament, umfassend mindestens ein festes Teil und einen im Verhältnis zum festen Teil in vertikaler Richtung verschiebbaren Wagen, wobei der Wagen mit einer horizontal herausragenden Welle versehen ist, mit der die Ausrüstung verbunden und um deren Zentrumachse die Ausrüstung drehbar ist, wobei eine erste Heb- und Drehvorrichtung, an deren Welle eine Scheibe angebracht ist, wenigstens teilweise eine runde Peripherieoberfläche aufweist und bei'der die Scheibe mit der Ausrüstung fest verbunden ist.

Eine Röntgenuntersuchungsausrüstung dieser Art ist aus dem Prospekt "Der neue Maßstab für Multifunktionsanlagen POLYSTAR T.O.P." der Fa. Siemens, Bestell-Nr. A91100-M1170-E250-01, Druckzeichen BKW 61250 WS 02973, erschienen im Februar 1997, bekannt. Die herausragende Welle ist mit einem Schneckenantrieb versehen, der seinerseits über einen Balken mit der Ausrüstung fest verbunden ist. Die Ausrüstung umfaßt den Balken, einen Patiententisch, ein C-Bogenstativ mit einer Röntgenröhre und einen Rezeptor, wobei der Patiententisch und das Stativ am Balken angebracht und unabhängig voneinander an diesem verschiebbar angeordnet sind. Der im Fundament angeordnete Wagen und damit auch der Balken sind mit Hilfe einer mittels eines Motors angetriebenen Gewindeschraube in vertikaler Richtung verschiebbar. Der Balken ist mit Hilfe des Schneckenantriebes um die Zentrumachse des Schneckenantriebes drehbar. Wenn der Schwerpunkt der Ausrüstung in Verbindung mit einer Untersuchung entlang dem Balken verschoben wird, ist ein Schneckenantrieb mit genauen Toleranzen erforderlich, so daß lediglich sehr kleine Spiele zwischen den Gewinden der Schneckenschraube und den Zahnen des Schneckenrades vorhanden sind. Nur so kann eine derartige Stabilität in der Ausrüstung erreicht werden, daß überraschende Rucke oder Winkeländerungen des Balkens vermieden werden können. Schneckenantriebe dieser Art sind extrem teuer. Auch die Gewindeschraube und der Motor für die vertikale Verschiebung des Wagens müssen so dimensioniert sein, daß sie ohne Probleme die Ausrüstung heben und senken können. Die besagte Konstruktion ist insgesamt aufwendig und außerdem in der Herstellung teuer.

In der FR 2 581 856 A1 ist ebenfalls eine derartige Röntgendiagnostikeinrichtung mit einem festen Teil und einem im Verhältnis zum festen Teil in vertikaler Richtung mittels einer Heb- und Drehvorrichtung verschiebbaren Wagen beschrieben. Der Wagen ist mit einer horizontal herausragenden Welle versehen, die mit einem Patientenlagerungstisch verbunden ist. Um der Achse der Welle ist der Patientenlagerungstisch mittels eines Transmissionsantriebes mit Zahnrädern drehbar angeordnet.

Der Erfindung liegt die Aufgabe zugrunde, eine Heb- und Drehvorrichtung für eine Röntgenuntersuchungsausrüstung der eingangs genannten Art zu schaffen, die in der Herstellung Verhältnismäßig billig ist und die außerdem solche Eigenschaften aufweist, daß die Ausrüstung auch bei einer Verschiebung des Schwerpunktes stabil ist.

Die Aufgabe wird erfindungsgemäß dadurch gelöst, daß mindestens ein erstes Paar von Rädern an dem festen Teil des Fundaments angeordnet ist und daß ein Verbindungsmittel dazu vorgesehen ist, die Scheibe mit den Rädern derart zu verbinden, daß, wenn jedes Rad des ersten Paares von Rädern gleichzeitig in verschiedenen Richtungen gedreht wird, die Scheibe und damit die Ausrüstung beeinflußt werden, sich in vertikaler Richtung zu verschieben, und wenn die Räder des ersten Paares von Rädern in dieselbe Richtung gedreht werden, die Scheibe und damit die Ausrüstung beeinflußt werden, sich um die Zentrumachse zu drehen. Hierdurch wird eine im Aufbau Verhältnismäßig einfache und damit in der Herstellung billige und gleichzeitig eine verhältnismäßig stabile Heb- und Drehvorrichtung erzielt.

In einer vorteilhaften Weiterentwicklung der Heb- und Drehvorrichtung nach der Erfindung wird ein zweites Paar von Rädern vorgeschlagen, das am Wagen oder an dem festen Teil des Fundaments angeordnet ist, und daß das Verbindungsmittel zunächst das eine Rad des ersten Paares der Räder teilweise umschließt, und danach das eine Rad des zweiten Paares der Räder teilweise umschließt, danach die Scheibe teilweise umschließt, danach das zweite Rad des zweiten Paares der Räder teilweise umschlie8t, und danach das zweite Rad des ersten Paares der Räder teilweise umschließt, wobei das erste und das zweite Paar von Rädern in verschiedenen Ebenen angeordnet sind. Mit Hilfe des zweiten Paares von Rädern und dem beschriebenen Weg des Verbindungsmittels in der Heb- und Drehvorrichtungskonstruktion ist eine weitere Stabilisierung der Vorrichtung gegeben.

Die Räder des ersten Paares von Rädern sind nach der Erfindung Antriebsmittel und die Räder des zweiten Paares von Rädern sind Umlenkräder.

Das Verbindungsmittel kann vorzugsweise eine Kette, aber auch ein Zahnriemen sein.

In Verbindung mit einer weiteren vorteilhaften Weiterentwicklung der Erfindung wird eine zweite Heb- und Drehvorrichtung vorgeschlagen, die mit der ersten Heb- und Drehvorrichtung parallel angeordnet ist. Dies ist eine Sicherheitsmaßnahme, die ein stetes Funktionieren der Vorrichtung gewährleistet.

Die Erfindung ist nachfolgend anhand mehrerer in den Zeichnungen dargestellter Ausführungsbeispiele näher erläutert. Es zeigen:
- FIG. 1: eine Röntgenuntersuchungsausrüstung mit einem Fundament, in dem eine Heb- und Drehvorrichtung nach der Erfindung angebracht werden kann.
- FIG. 2: ein Fundament nach der FIG. 1 ohne Umhüllung mit einer Heb- und Drehvorrichtung nach der Erfindung.
- FIG. 3: ein Fundament nach der FIG. 2 mit der Heb- und Drehvorrichtung in einer weiteren Lage als die, die in der FIG. 2 gezeigt ist.
- FIG. 4: schematisch eine zweite Ausführungsform einer Heb- und Drehvorrichtung nach der Erfindung.
- FIG. 5: schematisch eine dritte Ausführungsform einer Heb- und Drehvorrichtung nach der Erfindung und
- FIG. 6: schematisch eine vierte Ausführungsform einer Heb- und Drehvorrichtung nach der Erfindung.

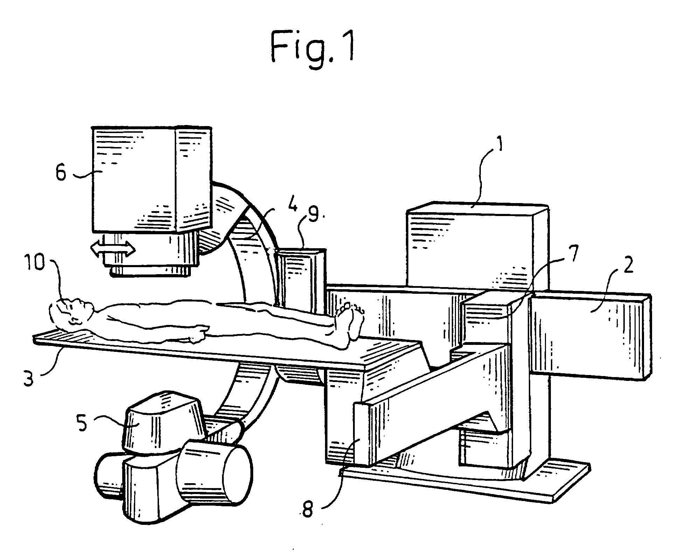

In der FIG. 1 ist eine Röntgenuntersuchungsausrüstung mit einem Fundament 1 gezeigt, bei der die Ausrüstung einen am Fundament 1 angebrachten Balken, einen Patiententisch 3 und ein C-Bogenstativ 4 mit einer Röntgenröhre 5 und einen Rezeptor 6 umfaßt. Der Patiententisch 3 ist über einen Träger 7 und einen Ausleger 8 mit dem Balken verbunden, wobei der Träger 7 und damit auch der Tisch 3 am Balken 2 entlang verschiebbar angeordnet ist. Auch das Stativ 4 ist über einen weiteren Träger 9 mit dem Balken 2 verbunden und ist an diesem entlang verschiebbar. Der in der Figur gezeigte Patient hat das Bezugszeichen 10. Der Balken 2 und damit die ganze Ausrüstung ist mittels einer Heb- und Drehvorrichtung in der Höhe verschiebbar und außerdem um eine hier nicht gezeigte aber später beschriebenen Welle drehbar.

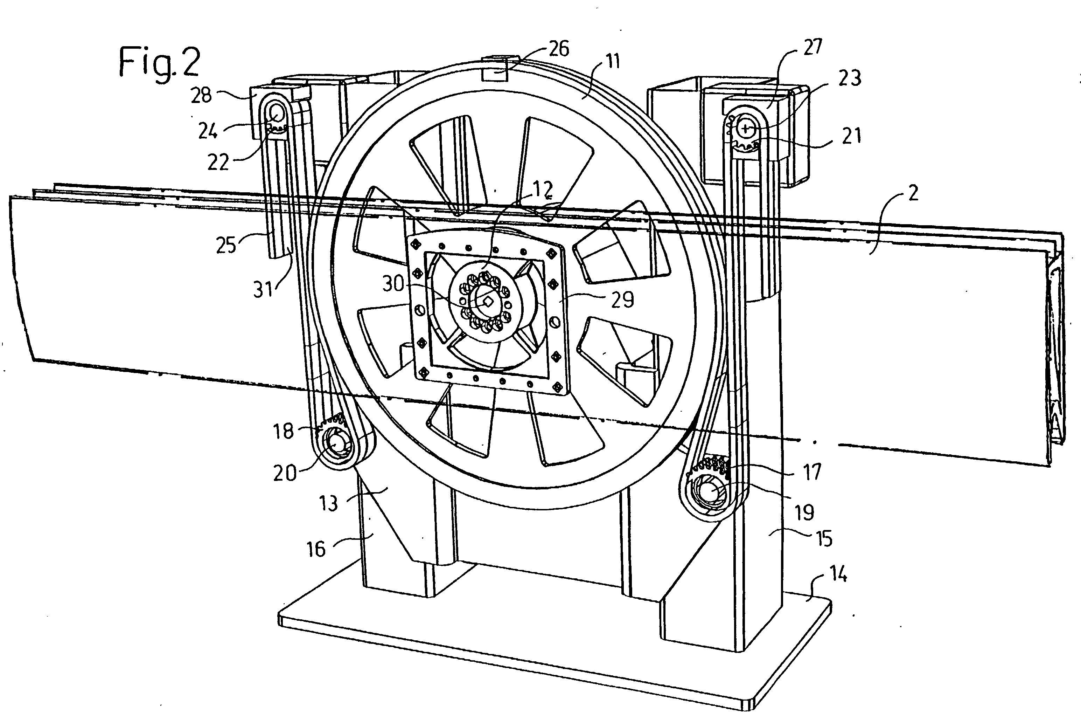

In der FIG. 2 ist das Fundament 2 ohne Umhüllung gezeigt. Hierdurch ist die Heb- und Drehvorrichtung freigelegt worden. Die Vorrichtung umfaßt eine radförmige Scheibe 11, die über eine Welle 12 mit einem Wagen 13 verbunden ist. Der Wagen 13 ist mittels bekannter und daher nicht näher gezeigter Mittel zwischen zwei in der Fundamentplatte 14 fest angeordneten Säulen 15, 16 in vertikaler Richtung verschiebbar. Die Vorrichtung umfaßt auch ein Paar von Antriebsrädern 21, 22, die an jeweils einer Säule 15, 16 angebracht und die mit hier nicht gezeigten Motoren über jeweils eine Welle 23, 24 verbunden sind. Die Vorrichtung umfaßt weiter ein Paar von Umlenkrädern 17, 18, die am Wagen 13 befestigt und um die Wellen 19, 20 drehbar sind. Ein Verbindungsmittel 25 in Form einer Kette verbindet die Paare von Rädern 17, 18, 21, 22 mit der Scheibe 11. So umschließt das Verbindungsmittel 25 zunächst teilweise das Antriebsrad 22 und danach teilweise das Umlenkrad 18, danach teilweise die Scheibe 11, danach teilweise das Umlenkrad 17 und schließlich umschließt die Kette 25 teilweise das Antriebsrad 21. Die Figur zeigt, daß die Kette 25 über eine Klammer 26 mit der Scheibe 11 fest verbunden sein kann. Es sind Führungsklötze 25 vorhanden, die zusehen sollen, daß die Kette 25 von den Antriebsrädern 21, 22 nicht gelöst wird. Der Balken 2, der in der Figur mit strichpunktierten Linien angedeutet ist, ist über ein mit einem Flansch versehenes Teil 29 mit der Scheibe 11 fest verbunden.

Mit Hilfe des beschriebenen Aufbaus der Heb- und Drehvorrichtung können nun mittels der Antriebsräder 21, 22 die Scheibe 11 bzw. der Balken 2 und damit die Ausrüstung dazu gebracht werden, in eine vertikale Richtung verschoben zu werden, getrennt um die Welle 12 gedreht zu werden oder dazu gebracht werden, eine kombinierte, kontinuierliche Heb- und Drehbewegung vorzunehmen. Eine Bewegung in vertikaler Richtung ist gegeben, wenn die Antriebsräder 21, 22 mittels der Motoren dazu gebracht werden, gleichzeitig in entgegengesetzten Richtungen zu drehen. Hierdurch werden durch die Kette 25 die Umlenkräder 17, 18 und damit der Wagen 13 gezwungen, entlang der Säulen 15, 16 verschoben zu werden. Wenn die Antriebsräder 21, 22 dazu gebracht werden, in dieselbe Richtung zu drehen, werden die Scheibe 11 bzw. der Balken und damit die Ausrüstung mit Hilfe der Kette 25 beeinflußt, um die Achse 30 der Welle 12 zu drehen. Die Motoren für die Antriebsräder 21, 22 sind vorzugsweise Teile eines bekannten und daher nicht näher beschriebenen softwaregesteuerten Steuersystems. Bei einer kombinierten Heb- und Drehbewegung des Balkens 2 wird die Steuerung derart softwarekontrolliert, daß eine kontinuierliche und weiche Bewegung der Ausrüstung gegeben ist.

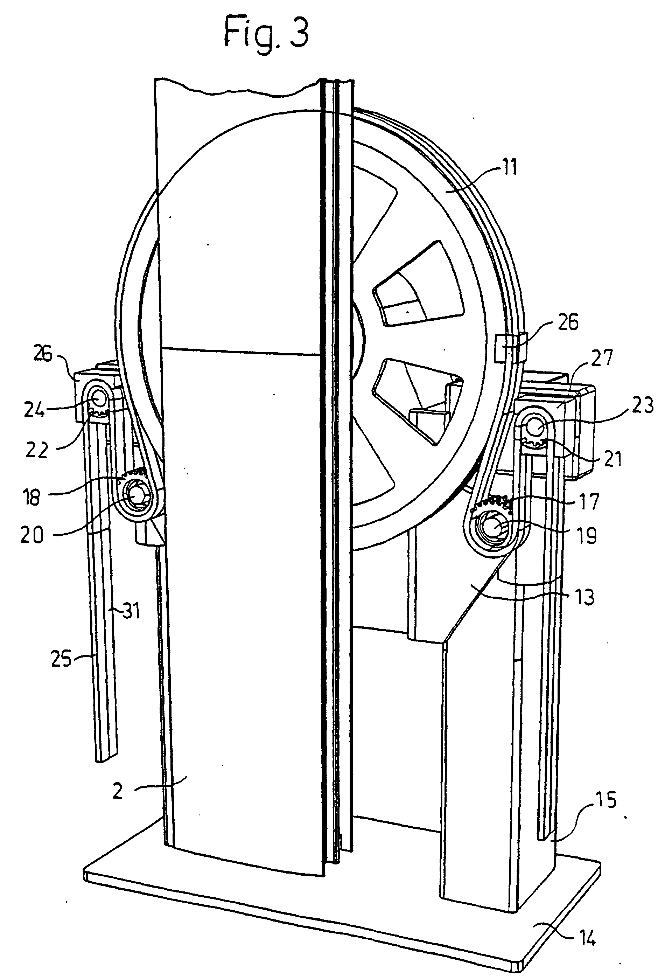

In der FIG. 3 ist eine Lage gezeigt, in der die Scheibe 11 bzw. der Balken 2 in beschriebener Weise mit Hilfe der Antriebsräder 21, 22 und der Motoren des erwähnten Steuersystems wechselweise in eine Position gehoben und gedreht worden sind, in der der Balken 2 eine vertikale Lage eingenommen hat.

In den FIG. 1 und 2 ist gezeigt, daß die Antriebs- und Umlenkräder 21, 22, 17, 18 sowie die Scheibe 11 zwei Peripherieoberflächen aufweisen, die erlauben, daß eine weitere Kette 31 parallel mit der genannten Kette 25 verlaufen kann. Diese weitere Kette 31 dient als Sicherheitsmaßnahme und als Verstärkung der Konstruktion.

Die Scheibe weist einen verhältnismäßig großen Durchmesser auf. Solange sich der Schwerpunkt der Ausrüstung im Bereich der Peripherie der Scheibe 11 befindet, ist eine besonders stabile Heb- und Drehvorrichtung gegeben. Eine Regel des Schwerpunktes, d.h. eine derartige Verschiebung des Patiententisches 3 und des C-Bogenstativs 4 entlang des Balkens 2, daß der Schwerpunkt sich innerhalb der Peripherie der Scheibe 11 befindet, kann softwarekontrolliert werden.

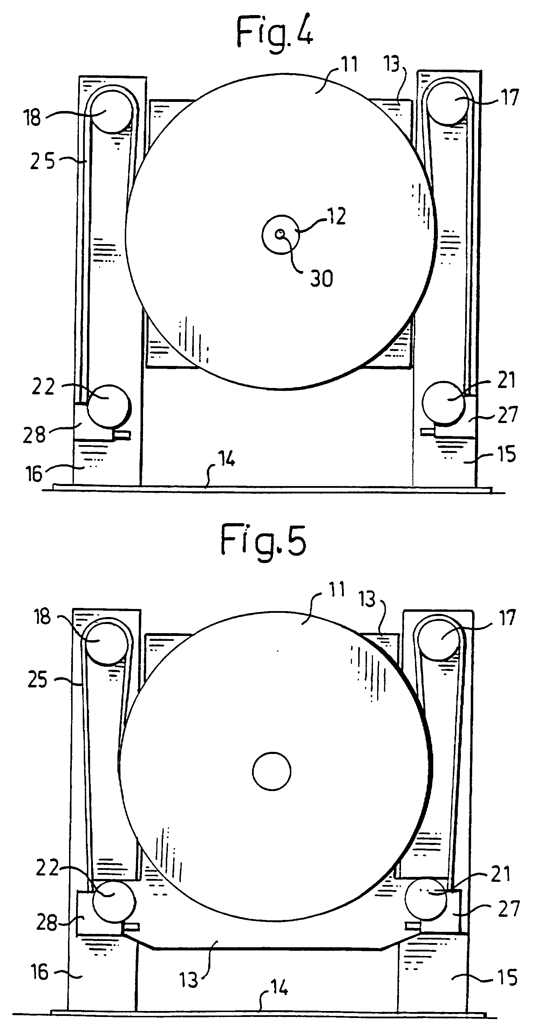

In der FIG. 4 ist eine weitere Ausführungsform der Heb- und Drehvorrichtung nach der Erfindung schematisch gezeigt. Die Bezugszeichen sind dieselben wie in den FIG. 1 und 2. In dem hier gezeigten Aufbau der Heb- und Drehvorrichtung sind sowohl das Paar von Antriebsrädern 21, 22 als auch das Paar von Umlenkrädern 17, 18 an der jeweiligen Säule 15, 16 angebracht, wobei das Paar von Umlenkrädern 17, 18 in diesem Beispiel in einer Ebene oberhalb des Paares von Antriebsrädern 21, 22 angeordnet ist. Hierdurch wird im Gegensatz zu dem Ausführungsbeispiel, das in den FIG. 1 und 2 gezeigt worden ist, die Scheibe 11 von der Kette 25 teilweise von unten umschlossen. Es sind Führungsklötze 27, 28 vorgesehen, die vorzugsweise so appliziert werden, daß die Kette 25 sich von den Antriebsrädern 21, 22 nicht lösen.

In der FIG. 5 ist gezeigt, daß das Paar von Antriebsrädern 21, 22 auch am Wagen angebracht werden kann. Im übrigen arbeitet die Heb- und Drehvorrichtung in der FIG. 4 und 5 wie es in Verbindung mit den FIG. 1 und 2 ausführlich beschrieben worden ist.

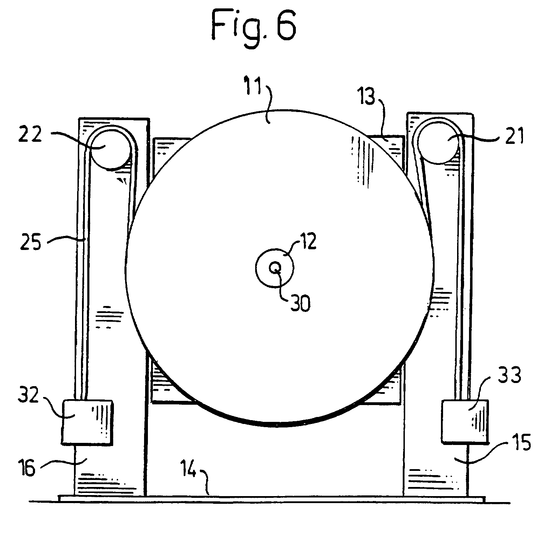

In der FIG. 6 ist eine Heb- und Drehvorrichtung nach der Erfindung mit einem verhältnismäßig einfachen Aufbau gezeigt. In diesem Ausführungsbeispiel umschließt die Kette 25 zunächst teilweise das eine Antriebsrad 22 und danach teilweise die Scheibe 11 und schließlich teilweise das zweite Antriebsrad 21. Es sind in diesem Ausführungsbeispiel keine Umlenkräder vorgesehen. Die Enden der Kette 25 werden hier in Behältern 32, 33 aufgesammelt. Auch dieser Aufbau der Heb- und Drehvorrichtung nach der Erfindung funktioniert, wie es in Verbindung mit den FIG. 1 und 2 beschrieben worden ist.

Die in Verbindung mit den FIG. 1 bis 6 beschriebene Kette 25 kann durch einen Zahnriemen ersetzt werden. Die Enden der Ketten bzw. der Zahnriemen, die in den FIG. 2 bis 5 gezeigt worden sind, werden auch in Schächten oder Behältern der in der FIG. 6 genannten Art aufgesammelt.

Die Peripherieoberfläche der Scheibe 11 braucht lediglich im Bereich derjenigen Zirkelsegmente, in denen die Kette oder der Zahnriemen gegen die Peripherieoberfläche anliegen, eine runde Form aufweisen.

Eine Heb- und Drehvorrichtung nach der Erfindung kann vorzugsweise auch in Verbindung mit einem Fundament, das nur einen Untersuchungstisch trägt, verwendet werden.

### Bezugszeichenliste

- 1: Fundament
- 2: Balken
- 3: Patiententisch
- 4: C-Bogenstativ
- 5: Röntgenröhre
- 6: Rezeptor
- 7, 9: Träger
- 8: Ausleger
- 10: Patient
- 11: Scheibe
- 12, 19, 20, 23, 24: Welle
- 13: Wagen
- 14: Fundamentplatte
- 15, 16: Säule
- 17, 18: Paar von Umlenkrädern, Umlenkrad
- 21, 22: Paar von Antriebsrädern, Antriebsrad
- 25: Verbindungsmittel, Kette
- 26: Klammer
- 27, 28: Führungsklotz
- 29: Teil
- 30: Achse
- 31: Kette
- 32,33: Behälter

## Patentansprüche

1. Röntgenuntersuchungsausrüstung mit einem Fundament (1), umfassend mindestens ein festes Teil (15, 16) und einen im Verhältnis zum festen Tell (15, 16) in vertikaler Richtung verschiebbaren Wagen (13), wobei der Wagen (13) mit einer horizontal herausragenden Welle (12) versehen ist, mit der die Ausrüstung (2, 3, 4) verbunden und um deren Zentrumachse (30) die Ausrüstung (2, 3, 4) drehbar ist, wobei eine erste Heb- und Drehvorrichtung (11, 17, 18, 21, 22, 25), an deren Welle (12) eine Scheibe (11) angebracht ist, wenigstens teilweise eine runde Peripherieoberfläche aufweist und bei der die Scheibe (11) mit der Ausrüstung (2, 3, 4) fest verbunden ist, **dadurch gekennzeichnet, daß** mindestens ein erstes Paar von Rädern (21, 22) an dem festen Teil (15, 16) des Fundaments (1) angeordnet ist und daß ein Verbindungsmittel (25) dazu vorgesehen ist, die Scheibe (11) mit den Rädern (21, 22) derart zu verbinden, daß, wenn jedes Rad (21, 22) des ersten Paares von Rädern (21, 22) gleichzeitig in verschiedenen Richtungen gedreht wird, die Scheibe (11) und damit die Ausrüstung (2, 3, 4) beeinflußt wird, sich in vertikaler Richtung zu verschieben, und wenn die Räder (21, 22) des ersten Paares von Rädern (21, 22) in dieselbe Richtung gedreht werden, die Scheibe (11) und damit die Ausrüstung (2, 3, 4) beeinflußt werden, sich um die Zentrumachse (30) zu drehen.

2. Röntgenuntersuchungsausrüstung nach Anspruch 1 **gekennzeichnet durch** ein zweites Paar von Rädern (17, 18), das am Wagen (13) oder an dem festen Teil (15, 16) des Fundaments (1) angeordnet ist, und daß das Verbindungsmittel (25) zunächst das eine Rad (22) des ersten Paares der Räder (21, 22) teilweise umschließt, und danach das eine Rad (18) des zweiten Paares der Räder (17, 18) teilweise umschließt, danach die Scheibe (11) teilweise umschließt, danach das zweite Rad (17) des zweiten Paares von Rädern (17, 18) teilweise umschließt und danach das zweite Rad (21) des ersten Paares von Rädern (21, 22) teilweise umschließt, wobei das erste und das zweite Paar von Rädern (21, 22, 17, 18) in verschiedenen Ebenen angeordnet sind.

3. Röntgenuntersuchungsausrüstung nach Anspruch 1 oder 2, **dadurch gekennzeichnet, daß** die Räder (17, 18) des ersten Paares von Rädern (17, 18) Antriebsmittel sind.

4. Röntgenuntersuchungsausrüstung nach einem der Ansprüche 1 bis 3, **dadurch gekennzeichnet, daß** die Räder (21, 22) des zweiten Paares von Rädern (21, 22) Umlenkräder sind.

5. Röntgenuntersuchungsausrüstung nach einem der Ansprüche 1 bis 4, **dadurch gekennzeichnet, daß** das Verbindungsmittel (25) eine Kette ist.

6. Röntgenuntersuchungsausrüstung nach einem der Ansprüche 1 bis 4, **dadurch gekennzeichnet, daß** das Verbindungsmittel (25) ein Zahnriemen ist.

7. Röntgenuntersuchungsausrüstung nach einem der Ansprüche 1 bis 6, **gekennzeichnet durch** eine zweite Heb- und Drehvorrichtung (11, 17, 18, 21, 22, 31), die mit der ersten Heb- und Drehvorrichtung (11, 17, 18, 21, 22, 25) parallel angeordnet ist.

## Claims

1. An X-ray examination equipment having a base (1), comprising at least one fixed part (15, 16) and a carriage (13) which can be moved vertically in relation to the fixed part (15, 16), the carriage (13) being provided with a horizontally projecting shaft (12) to which the equipment (2, 3, 4) is connected and about the centre axis of which the equipment (2, 3, 4) can rotate, a first lifting and rotating device (11, 17, 18, 21, 22, 25) on the shaft (12) of which a pulley (11) is mounted having at least partially a round peripheral surface and in which the pulley (11) is securely connected to the equipment (2, 3, 4),
**characterised in that** at least one first pair of wheels (21, 22) is arranged at the fixed part (15, 16) of the base (1) and that a connecting means (25) is provided in order to connect the pulley (11) to the wheels (21, 22) in such a way that whenever each wheel (21, 22) of the first pair of wheels (21, 22) is simultaneously turned in a different direction, the pulley (11) and hence the equipment (2, 3, 4) are caused to move in a vertical direction and whenever the wheels (21, 22) of the first pair of wheels (21, 22) are turned in the same direction, the pulley (11) and hence the equipment (2, 3, 4) are caused to rotate about the centre axis (30).

2. The X-ray examination equipment according to claim 1, **characterised by** a second pair of wheels (17, 18) which is arranged on the carriage (13) or on the fixed part (15, 16) of the base (1) and in that initially the connecting means (25) partially covers one wheel (22) of the first pair of wheels (21, 22) and subsequently partially covers one wheel (18) of the second pair of wheels (17, 18), then partially covers the pulley (11), then partially covers the second wheel (17) of the second pair of wheels (17, 18), and then partially covers the second wheel (21) of the first pair of wheels (21, 22), the first pair of wheels and the second pair of wheels (21, 22, 17, 18) being arranged in different planes.

3. The X-ray examination equipment according to claim 1 or 2,
**characterised in that** the wheels (17, 18) of the first pair of wheels (17, 18) are driving means.

4. The X-ray examination equipment according to one of claims 1 to 3,
**characterised in that** the wheels (21, 22) of the second pair of wheels (21, 22) are deflection wheels.

5. The X-ray examination equipment according to one of claims 1 to 4,
**characterised in that** the connecting means (25) is a chain.

6. The X-ray examination equipment according to one of claims 1 to 4,
**characterised in that** the connecting means (25) is a toothed belt.

7. The X-ray examination equipment according to one of claims 1 to 6,
**characterised by** a second lifting and rotating device (11, 17, 18, 21, 22, 31) which is arranged parallel to the first lifting and rotating device (11, 17, 18, 21, 22, 31).

## Revendications

1. Equipement d'analyse radiographique, comportant une base (1) comprenant au moins une partie (15, 16) fixe et un chariot (13) mobile en direction verticale par rapport à la partie (15, 16) fixe, le chariot (13) étant pourvu d'un arbre (12) dépassant horizontalement, arbre sur lequel l'équipement (2, 3, 4) est monté à rotation autour de l'axe (30) central de cet arbre, un premier dispositif (11, 17, 18, 21, 22, 25) de levage et de rotation, sur l'arbre (12) duquel est monté une poulie (11), ayant une surface périphérique au moins partiellement circulaire, et la poulie (11) étant fixement assemblée à l'équipement (2, 3, 4), **caractérisé en ce qu'**au moins une première paire (21, 22) de roues est disposée sur la partie (15, 16) fixe de la base (1), et **en ce qu'**un moyen (25) de liaison est prévu pour relier la poulie (11) aux roues (21, 22) de telle sorte que, lorsque les roues (21, 22) de la première paire (21, 22) de roues sont simultanément mises en rotation dans des sens différents, la poulie (11) et donc l'équipement (2, 3, 4) sont conduits à se déplacer en direction verticale, et que, lorsque les roues (21, 22) de la première paire (21, 22) de roues sont mises en rotation dans le même sens, la poulie (11) et donc l'équipement (2, 3, 4) sont conduits à tourner autour de l'axe (30) central.

2. Equipement d'analyse radiographique suivant la revendication 1, **caractérisé par** une deuxième paire (17, 18) de roues, qui est disposée sur le chariot (13) ou sur la partie (15, 16) fixe de la base (1), et par le fait que le moyen (25) de liaison entoure d'abord partiellement l'une (22) des roues de la première paire (21, 22) de roues, puis entoure partiellement l'une (18) des roues de la deuxième paire (17, 18) de roues, puis entoure partiellement la poulie (11), puis entoure partiellement la deuxième roue (17) de la deuxième paire (17, 18) de roues, puis entoure partiellement la deuxième roue (21) de la première paire (21, 22) de roues, la première paire (21, 22) de roues et la deuxième paire (17, 18) de roues étant disposées dans des plans différents.

3. Equipement d'analyse radiographique suivant la revendication 1 ou 2, **caractérisé en ce que** les roues (21, 22) de la première paire (21, 22) de roues sont des moyens d'entraînement.

4. Equipement d'analyse radiographique suivant l'une des revendications 1 à 3, **caractérisé en ce que** les roues (17, 18) de la deuxième paire (17, 18) de roues sont des roues de renvoi.

5. Equipement d'analyse radiographique suivant l'une des revendications 1 à 4, **caractérisé en ce que** le moyen (25) de liaison est une chaîne.

6. Equipement d'analyse radiographique suivant l'une des revendications 1 à 4, **caractérisé en ce que** le moyen (25) de liaison est une courroie dentée.

7. Equipement d'analyse radiographique suivant l'une des revendications 1 à 6, **caractérisé par** un deuxième dispositif (11, 17, 18, 21, 22, 31) de levage et de rotation, qui est disposé en parallèle au premier dispositif (11, 17, 18, 21, 22, 25) de levage et de rotation.
